# EUROPEAN PATENT SPECIFICATION

(11) **EP 4 069 341 B1**
(45) Date of publication and mention of the grant of the patent: **30.07.2025**
(21) Application number: 20825163.7
(22) Date of filing: 27.11.2020
(51) Int. Cl.: A61M 5/32, A61M 5/28

(54) **BLINDING CAP**
VERBLINDUNGSKAPPE
CAPUCHON PROTECTEUR

(30) Priority: 04.12.2019 GB 201917728; 30.03.2020 GB 202004619; 25.09.2020 GB 202015182
(43) Date of publication of application: 12.10.2022
(73) Proprietor: Almac Clinical Services Limited, Craigavon County Armagh BT63 5QD (GB)
(72) Inventor: CARTWRIGHT, Jonathan, Craigavon Northern Ireland BT63 5QD (GB)
(74) Representative: Boult Wade Tennant LLP
(86) International application number: PCT/GB2020/053029
(87) International publication number: WO 2021/111110

(56) References cited:
- EP-A1- 3 524 300
- WO-A1-2019/074788
- KR-A- 20160 092 813

## Description

The present disclosure relates to a blinding cap, a syringe assembly comprising a blinding cap and a method of assembling a syringe assembly.

### Background to the disclosure

Syringes are widely used for the administration of fluids, typically liquids. A syringe functions as a pump and typically comprises a piston that is moveable within a barrel to expel fluid stored in the barrel out of an outlet of the barrel. The outlet of the barrel may communicate with a needle, such as a hypodermic needle. To prevent needle-stick injury during storage and transport of the syringe it is common to provide a rigid needle shield that is releasably mounted on the syringe to cover the needle prior to use.

Syringes may be provided with identification markings containing information such as the type of fluid (e.g. medicament) in the syringe, brand name(s), manufacturer details, lot and or batch numbers, marketing authorisation details, or product licence details. In clinical trial settings, syringes may be provided with identification markings containing other information such as trial number, lot number, group number, or similar to allow blinded studies to be carried out. In a 'blinded' study it is important to ensure that the patient (also known as the trial participant) and also the user of the syringe (e.g. a doctor, nurse, or potentially the patient themselves) do not know the full details of what is being administered. In one example, it may be important to ensure that a patient and a user of the syringe are unaware whether a syringe contains an active medicament or a placebo. This may include obscuring any identification markings provided on the rigid needle shield.

EP3524300 describes a blinding cap for covering a rigid needle shield that is covering a needle of a syringe and includes an elongated body extending between a proximal end and an opposing distal end, the body at least partially bounding a chamber. An annular sleeve can project from the proximal end of the body and at least partially bound a channel. A first flange radially inwardly projects from the body into the chamber, the first flange at least partially bounding an opening that provides communication between the chamber and the channel. The elongated body is comprised of first and second cap portions.

In light of this background there is still a need to provide improvements in the blinding of parts of syringes, especially when such syringes are to be used in clinical trials where it is important that the user and or patient cannot readily identify the contents of the syringe. For example, there is a need to provide blinding caps that are more robust while being easier to assemble with the syringe.

### Summary of the disclosure

According to a first aspect of the present disclosure there is provided a blinding cap for covering a rigid needle shield that is releasably mounted on a syringe, the blinding cap comprising a first part and a second part joined together by a hinge;
the first part comprising:
   - a first elongate bowl-shaped body defining a first recess;
   - a first flange portion located at a proximal end;
   - a first rim extending at least partially around an open mouth of the first elongate bowl-shaped body;
   - a raised lip extending at least partially along the first rim;
   - one or more fins that project inwardly from the first elongate bowl-shaped body; and
   - a first locking member;
the second part comprising:
   - a second elongate bowl-shaped body defining a second recess;
   - a second flange portion located at a proximal end;
   - a second rim extending at least partially around an open mouth of the second elongate bowl-shaped body;
   - a recess extending at least partially along the second rim;
   - one or more fins that project inwardly from the second elongate bowl-shaped body; and
   - a second locking member;
wherein the blinding cap is configured such that the first part and the second part can be brought into engagement around the rigid needle shield by flexure of the hinge such that:
   i) the raised lip of the first part is engaged in the recess of the second part;
   ii) the first locking member and the second locking member engage one another to lock the first part and the second part together;
   iii) the first recess and second recess together define a cavity for receiving the rigid needle shield of the syringe;
   iv) the first flange portion and the second flange portion are juxtaposed to form an inwardly-projecting flange that is configured to be disposed between an end surface of the rigid needle shield and a barrel of the syringe; and
   v) the one or more fins of the first part and the second part are configured to engage the rigid needle shield.

Advantageously, the blinding cap of the present disclosure provides for easy manual-handling of the blinding cap during blinding of a syringe.

Advantageously, the engagement of the raised lip in the recess may function not only to improve the robustness of the closed blinding cap but also to increase the security of the blinding cap by blinding any crack that might be present between the first rim and the second rim.

The one or more fins of the first part and the second part may together define a narrower bore within the cavity for engaging the rigid needle shield.

The one or more fins of the first part and the second part may comprise a first portion defining an end stop for an end face of the rigid needle shield and/or a second portion defining a side stop for a side face of the rigid needle shield and/or a corner portion defining a corner stop for a corner of the rigid needle shield.

The one or more fins of the first part and the second part may comprise one or more flexible fins or one or more rigid fins.

Advantageously, the one or more fins of the first part and the second part may act together to grip or restrain movement of at least a portion of the rigid needle shield to limit or prevent rattling or movement of the rigid needle shield within the blinding cap. This may improve user confidence by preventing a user being concerned that a part has broken or malfunctioned within the unseen interior of the blinding cap.

The inwardly-projecting flange may define a proximal opening at a proximal end.

The inwardly-projecting flange may comprise a chamfered edge.

A distal end of the first part and the second part may be rounded such that on engagement of the first part and the second part the blinding cap comprises a closed, rounded distal end.

The raised lip may extend around the rounded distal end of the first part and the recess may extend around the rounded distal end of the second part. This may prevent observation of the interior of the blinding cap by blinding any crack that might be present between the first rim and the second rim in the region of the rounded distal end.

The raised lip may extend along a lateral side of the first elongate bowl-shaped body proximate the hinge.

The first locking member may be provided on a lateral side of the first elongate bowl-shaped body opposite the hinge.

One of the first and second locking members may project above the first rim and the other of the first and second locking members may be recessed below the second rim.

The first locking member may comprise a locking frame comprises a plurality of struts extending from the first rim interconnected by one or more locking bars. The second locking member may comprise one or more locking tabs configured to interengage with the one or more locking bars by flexure of the struts.

The first part and the second part may be opaque.

The blinding cap may comprise a single unitary part. Alternatively, each of the first part and the second part may be single unitary parts. The hinge may be formed unitarily with the first part and/or the second part. Alternatively, the hinge may be separately formed and joined to the first part and the second part.

The first part and/or the second part and/or the blinding cap as a whole may be a moulded part and may optionally be formed of a rigid plastics material. Each is preferably opaque.

The first part and the second part may comprise a first half shell and a second half shell.

The hinge may be an elongate hinge. The hinge may extend over greater than 50%, optionally 70%, optionally 90% of the length of lateral side of the first elongate bowl-shaped body proximate the hinge. Beneficially such an elongate hinge may promote correct alignment of the first part and the second part during closure of the blinding cap, e.g. keeping the longitudinal axes of the first part and the second part parallel to one another. This may help to ease engagement of the first and second locking members.

The blinding cap may have a smooth exterior surface. For example, the blinding cap may comprise a cylindrical side wall and a rounded distal end so that it is free of abrupt discontinuities.

Advantageously, by configuring the blinding cap to have a smooth exterior surface the syringe assembly may be easier to package, in particular in a flow-wrap package.

According to a second aspect of the present disclosure there is provided a syringe assembly comprising a blinding cap according to any preceding claim and a syringe that comprises a barrel and a rigid needle shield that is releasably mounted over a needle of the syringe.

The syringe may be a manually-activated syringe.

According to a third aspect of the present disclosure there is provided a blinding cap for covering a rigid needle shield that is releasably mounted on a syringe, the blinding cap comprising:
a first collar section, a second collar section and a sleeve section which are initially separate from one another;
the first collar section and the second collar section being engagable against each other to form a collar unit for encircling the rigid needle shield of the syringe;
the collar unit being insertable into the sleeve section;
the collar unit comprising a first part of a securement and the sleeve section comprising a second part of the securement wherein on receipt of the collar unit within the sleeve section the first part and the second part of the securement engage one another to securely retain the collar unit within the sleeve section;
the collar unit comprising an inwardly-projecting flange that is configured to be disposed between an end surface of the rigid needle shield and a barrel of the syringe;
the collar unit and the sleeve section acting in combination for hindering observation of the rigid needle shield and for assisting in removal of the rigid needle shield from the syringe.

The present disclosure provides in a fourth aspect a syringe assembly comprising a blinding cap according to the third aspect described above and a syringe that comprises a barrel and a rigid needle shield that is releasably mounted over a needle of the syringe.

The present disclosure provides in a fifth aspect a method of assembling a syringe assembly from a plurality of separate components that comprise a first collar section, a second collar section, a sleeve section and a syringe that itself comprises a barrel and a rigid needle shield; the method comprising:
first encircling the rigid needle shield of the syringe by engaging the first collar section and the second collar section together to form a collar unit that encircles the rigid needle shield, the collar unit comprising an inwardly-projecting flange that is disposed between an end surface of the rigid needle shield and the barrel of the syringe;
subsequently inserting the collar unit and the encircled rigid needle shield into the sleeve section;
insertion of the collar unit into the sleeve section resulting in a first part of a securement, provided on the collar unit, and a second part of the securement, provided on the sleeve section, securely engaging one another to securely retain the collar unit within the sleeve section.

The present disclosure provides in a sixth aspect a method of preparing materials for carrying out a clinical trial, preferably a blinded clinical trial, which comprises assembling a plurality of syringe assemblies according to the second and or fourth aspects described above, wherein a first group of the plurality of syringe assemblies share a first characteristic and a second group of the plurality of syringe assemblies share a second, different characteristic. The characteristic may be, by way of example, one or more of: a type of medicament, a dosage of medicament, and the presence or absence of an active medicament - i.e. the first group may comprise an active medicament and the second group may comprise a placebo.

### Brief description of the drawings

One or more embodiments of the disclosure will now be described, by way of example only, with reference to the accompanying drawings in which:
Figure 1 is a perspective view of a first collar section and a second collar section of a first blinding cap useful for understanding the present disclosure;
Figure 2 is a perspective view of a sleeve section of the first blinding cap useful for understanding the present disclosure;
Figure 3 is a perspective view of the blinding cap of Figures 1 and 2 prior to assembly;
Figure 4 is a perspective view of the blinding cap of Figure 3 once assembled, but for clarity not showing a syringe received within the blinding cap;
Figure 5 is a schematic perspective view of a syringe;
Figure 6 is a schematic perspective view of a syringe assembly comprising the blinding cap of Figure 3 and the syringe of Figure 5;
Figure 7 is an exploded perspective view of a second blinding cap useful for understanding the present disclosure shown with a syringe;
Figure 8 is an exploded perspective view of the second blinding cap of Figure 7;
Figure 9 is a perspective view of a second collar section of the blinding cap of Figure 7;
Figure 10 is another perspective view of the second collar section of Figure 9;
Figures 11 and 12 are perspective views of a sleeve section of the blinding cap of Figure 7;
Figure 13 is a cross-sectional view of the sleeve section of the blinding cap of Figure 7;
Figure 14 is a perspective view of a variant of second collar section of the second blinding cap useful for understanding the present disclosure;
Figure 15 is another perspective view of the variant of the second collar section of Figure 14;
Figure 16 is a perspective view of a third embodiment of blinding cap according to the present disclosure in an open configuration;
Figure 17 is a perspective view of the blinding cap of Figure 16 together with a rigid needle shield;
Figure 18 is another perspective view of the blinding cap of Figure 16;
Figure 19 is a schematic view of a syringe attached to a rigid needle shield;
Figure 20 is a schematic view of a syringe assembly;
Figure 21 is a perspective view of a fourth embodiment of blinding cap according to the present disclosure in an open configuration;
Figure 22 is a top plan view of a syringe assembly comprising the blinding cap of Figure 21 together with a first design of rigid needle shield;
Figure 23 is a cross sectional view side elevation of the syringe assembly of Figure 22;
Figure 24 is an enlarged view of a portion of Figure 23;
Figure 25 is a perspective view of a fifth embodiment of blinding cap according to the present disclosure in an open configuration;
Figure 26 is a top plan view of a syringe assembly comprising the blinding cap of Figure 25 together with a second design of rigid needle shield;
Figure 27 is a cross sectional view side elevation of the syringe assembly of Figure 26;
Figure 28 is an enlarged view of a portion of Figure 27;
Figure 29 is a perspective view of a sixth embodiment of blinding cap according to the present disclosure in an open configuration;
Figure 30 is a top plan view of a syringe assembly comprising the blinding cap of Figure 29 together with a third design of rigid needle shield;
Figure 31 is a cross sectional view side elevation of the syringe assembly of Figure 30; and
Figure 32 is an enlarged view of a portion of Figure 31.

### Detailed Description

The following description is directed to one or more embodiments of the disclosure. The description of the embodiments is not meant to include all the possible embodiments of the disclosure that are claimed in the appended claims. Many modifications, improvements and equivalents which are not explicitly recited in the following embodiments may fall within the scope of the appended claims. Features described as part of one embodiment may be combined with features of one or more other embodiments unless the context clearly requires otherwise.

In this specification, the use of the singular includes the plural unless the context clearly dictates otherwise. In this application, the use of "or" means "and/or" unless stated otherwise. Furthermore, use of the term "including" as well as other forms, such as "include", "includes," and "included," is not limiting.

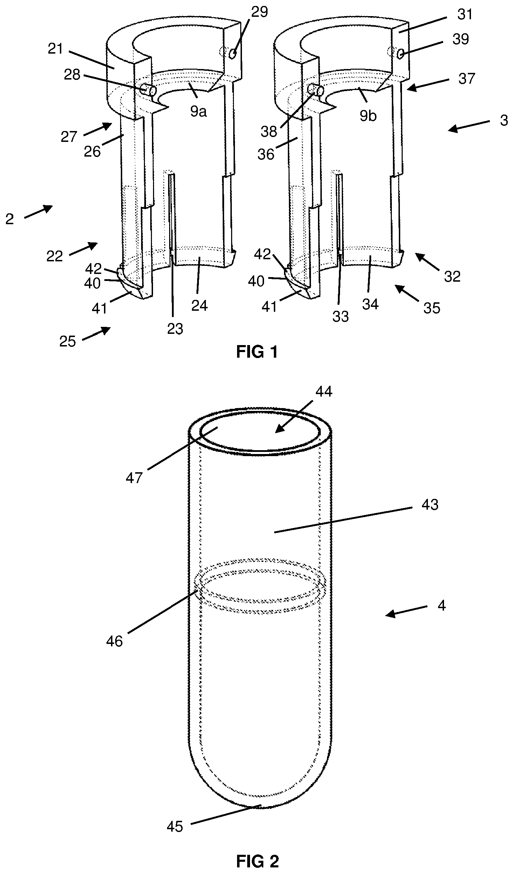

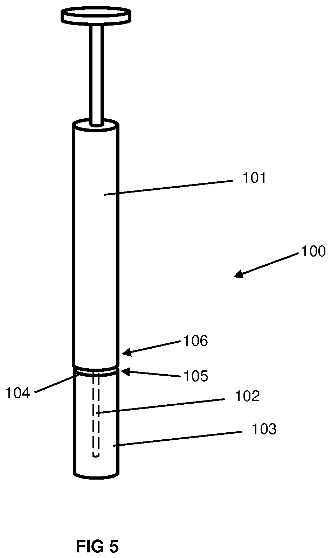

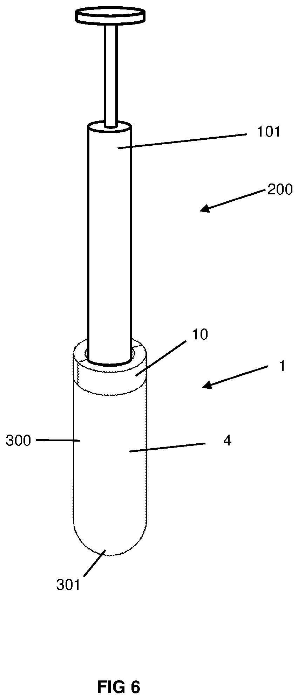

Figures 1 to 4 show a first blinding cap 1 useful for understanding the present disclosure. The blinding cap 1 is intended to be combined with a syringe 100, shown schematically in Figure 5, to form a syringe assembly 200, as shown schematically in Figure 6. The blinding cap 1 functions to cover a rigid needle shield 103 that is releasably mounted on a barrel 101 of the syringe 100 to shield a needle 102 of the syringe 100.

The blinding cap 1 comprises a first collar section 2 and a second collar section 3 as shown in Figure 1. The blinding cap 1 further comprises a sleeve section 4 as shown in Figure 2. Preferably, the blinding cap 1 is formed of only these three parts.

The first collar section 2, the second collar section 3 and the sleeve section 4 are initially separate from one another, i.e. they are separate components which are assembled together to form the blinding cap 1.

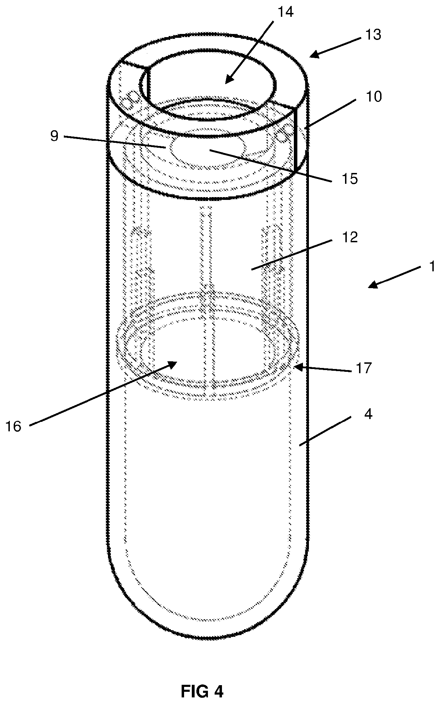

On assembly, the first collar section 2 and the second collar section 3 are engagable against each other to form a collar unit 10 for encircling the rigid needle shield 103 of the syringe 100 as shown in Figures 6. The collar unit 10 is insertable into the sleeve section 4 as shown in Figure 4.

The collar unit 10 comprises a first part of a securement and the sleeve section 4 comprises a second part of the securement. On receipt of the collar unit 10 within the sleeve section 4, the first part and the second part of the securement engage one another to securely retain the collar unit 10 within the sleeve section 4. The securement may comprise one or more sets of co-operating formations.

The collar unit 10 further comprises an inwardly-projecting flange 9, as shown in broken lines in Figure 4, that is configured to be disposed between an end surface 104 of the rigid needle shield and the barrel 101 of the syringe 100, which can be seen in Figure 5. An axial gap 105 may be provided between the end surface 104 and a distal end 106 of the barrel 101 to accommodate the inwardly-projecting flange 9.

The collar unit 10 and the sleeve section 4 act in combination to hinder observation of the rigid needle shield 103 and also for assisting in removal of the rigid needle shield 103 from the syringe 100 as discussed below.

As shown in Figure 1, the first collar section 2 and the second collar section 3 may each comprise a single unitary part. They may each be a moulded part. They may each be formed of a rigid plastics material. They may each be opaque. The first collar section 2 and the second collar section 3 may each comprise a half shell, namely a first half shell and a second half shell. The first collar section 2 and the second collar section 3 may be identical to each other.

The first collar section 2 may comprise a head 21 and a body 22. The body 22 may extend from a distal end of the head 21. Likewise, the second collar section 3 may comprise a head 31 and a body 32. The body 32 may extend from the distal end of the head 31.

Each of the heads 21, 31 may take a semi-annular shape, for example a semi-cylindrical shape. Each of the heads 21, 31 may comprise one or more alignment locators for aligning the first collar section 2 and the second collar section 3 with each other to maintain the shape of the collar unit 10 prior to insertion of the collar unit 10 into the sleeve section 4. In the example of Figure 1, the alignment locators comprise a projection 28, 38 and a recess 29, 39 that are co-operatively shaped and sized such that the projection 28 of the head 21 may be received in the recess 39 of the head 31 and the projection 38 of the head 31 may be received in the recess 29 of the head 21.

Each of the bodies 22, 32 may comprise an elongate extension. Each elongate extension may take a generally semi-annular shape, for example a semi-cylindrical shape. Each of the bodies 22, 32 may have a longitudinal length that is greater than a longitudinal length of the rigid needle shield 103. Each of the bodies 22, 32 may comprise one or more longitudinal slits 23, 33 that extend longitudinally along at least a part of the length of the bodies 22, 32 from a distal end 25, 35 of the bodies 22, 32 to thereby define two or more flexible tongues 24, 34 in each of the bodies 22, 32. The tongues 24, 34 may be cantilevered from an annular portion 26, 36 of the bodies 22, 32 which may be located at a proximal end 27, 37 of the bodies 22, 32. In the example of Figure 1, each body 22, 32 comprises three flexible tongues 24, 34 and two longitudinal slits 23, 33.

One or more or, but preferably all of, the flexible tongues 24, 34 may be provided with an outwardly-projecting lip 40 at or near their distal ends as shown in Figure 1. Each lip 40 may comprise a ramped surface 41 which is directed distally and a perpendicular face 42 located at a proximal end of the ramped surface 41. The first part of the securement may comprise or consist of the lips 40 and the associated flexible tongues 24, 34.

A first portion 9a of the inwardly-projecting flange 9 may be provided on the first collar section 2 and a second portion 9b of the inwardly-projecting flange 9 may be provided on the second collar section 3.

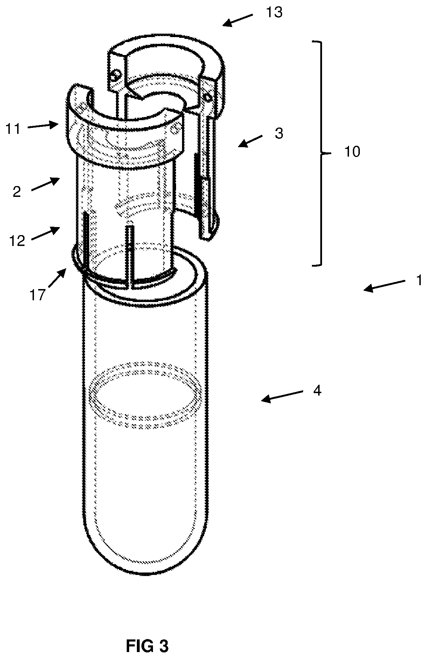

The head 21 of the first collar section 2 and the head 31 of the second collar section 3 when engaged next to each other may together form a head section 11 of the collar unit 10 as shown in Figure 3. The head section 11 may be annular in shape, for example a cylindrical shape. The head section 11 of the collar unit 10 may define a bore 14, as shown in Figure 4, for engaging and or accommodating the distal end 106 of the barrel 101.

The body 22 of the first collar section 2 and the body 32 of the second collar section 3 when engaged next to each other may together form a body section 12 of the collar unit 10. The body section 12 may have a longitudinal length that is greater than a longitudinal length of the rigid needle shield 103. Additionally or alternatively, the body section 12 may have an internal diameter that is greater than an external diameter of the rigid needle shield 103. The first portion 9a and the second portion 9b when engaged next to each other may together form an annular inwardly-projecting flange 9.

The inwardly-projecting flange 9 may be located at a proximal end 13 of the collar unit 10. Alternatively, the inwardly-projecting flange 9 may be located towards the proximal end 13 but with the bore 14 of the head section 11 extending proximally from the location of the inwardly-projecting flange 9 to the proximal end 13 as shown in Figure 4. The inwardly-projecting flange 9 may define, at its centre, a proximal opening 15 at or towards the proximal end 13 of the collar unit 10. The collar unit 10 may comprise a distal opening 16 at a distal end 17 of the collar unit 10. Once assembled, the distal end 17 of the collar unit 10 may comprise the plurality of flexible tongues 24, 34 of the first collar section 2 and the second collar section 3.

The sleeve section 4 may comprise a closed-ended tube as shown in Figure 2. The closed-end of the tube may be rounded. The sleeve section 4 may comprise a single unitary part. The sleeve section 4 may be a moulded part. The sleeve section 4 may be formed of a rigid plastics material. The sleeve section 4 is preferably opaque.

The sleeve section 4 may comprise a bore 43 for receiving the collar unit 10 that has an open first end 44 through which the collar unit 10 is received and a closed second end 45 opposite the open first end 44.

As shown in broken lines in Figure 2, the sleeve section 4 may comprise one or more recesses 46 for receiving the outwardly-projecting lips 40 of the flexible tongues 24, 34. In the example shown in Figure 2, one annular recess 46 is provided which extends around an inner face 47 of the bore 43 of the sleeve section 4. The second part of the securement may comprise or consist of the one or more recesses 46.

In use, to assemble the blinding cap 1 and form the syringe assembly 200, the first collar section 2 and the second collar section 3 are brought together about the syringe 100. In this step the rigid needle shield 103 is accommodated in the body section 12 of the collar unit 10 in between the bodies 22, 32. The inwardly-projecting flange 9 of the collar unit 10 is formed by the coming together of the first flange portion 9a and the second flange portion 9b so as to be located in the axial gap 105 between the end surface 104 of the rigid needle shield 103 and the distal end 106 of the barrel 101. The one or more projections 28 are received in the one or more recesses 29 to aid temporary alignment and retention of the first collar section 2 and the second collar section 3 on and about the rigid needle shield 103. The distal end 106 of the barrel 101 is received in the bore 14 of the head section 11.

Next, the body section 12 of the collar unit 10 may be inserted into the sleeve section 4. In this step the body section 12 passes through the open first end 44 into the bore 43. The distal end 17 of the collar unit 10 may be configured to deform to accommodate insertion of the collar unit 10 into the sleeve section 4. For example, the flexible tongues 24, 34 may flex inwardly due to engagement of the ramped surfaces 41 of the lips 40 engaging on a rim of the open first end 44. Inward flexure of the flexible tongues 24, 34 may be accommodated by a radial spacing between an internal face of the flexible tongues 24, 34 and an external face of the rigid needle shield 103. Additionally or alternatively, inward flexure of the flexible tongues 24, 34 may be accommodated by a longitudinal spacing between a distal end of the flexible tongues 24, 34 and a distal end of the rigid needle shield 103.

The collar unit 10 (and syringe 100) are slid into the bore 43 until the lips 40 reach the annular recess 46 at which point the flexible tongues 24, 34 resiliently flex back outwardly so as to engage the lips 40 in the annular recess 46. Preferably, at the same point of insertion the head section 11 is brought into contact against the proximal end of the sleeve section 4 without entering the bore 43 of the sleeve section 4 as shown in Figures 5 and 6. Preferably an external diameter of the head section 11 may be the same as the external diameter of the proximal end of the sleeve section 4. The inwardly-projecting flange 9 may be located longitudinally at an interface between the head section 11 and the body section 12.

Removal of the collar unit 10 from the sleeve section 4 is resisted by engagement of the perpendicular face 42 of the lips 40 against a proximal edge of the annular recess 46. Thus, the securement acts as a non-reversible means for connecting the collar unit 10 and the sleeve section 4.

The lips 40 and the flexible tongues 24, 34 are preferably fully received within the sleeve section 4 once the first part and the second part of the securement engage one another as shown in Figure 6. This effectively hinders attempts to tamper with the blinding cap 10.

Once the collar unit 10 is securely retained in the sleeve section 2 the rigid needle shield 103 is preferably fully enveloped by the blinding cap 1 except for the proximal opening 15 at or towards the proximal end 13 of the collar unit 10 defined by the inwardly-projecting flange 9. However, the proximal opening 15 is obscured by the presence of the distal end 106 of the barrel 101. Thus, a complete and effective blinding of the rigid needle shield 103 may be achieved.

As shown in Figure 6, the blinding cap 1 may have a smooth exterior surface. For example, the blinding cap 1 may comprise a cylindrical side wall 300 which may include the side walls of the sleeve section 4 and the collar unit 10 and a rounded distal end 301 as shown in Figure 6 so that it is free of abrupt discontinuities.

The syringe assembly 200 may be stored in packaging, for example a flow wrap packaging.

To use the syringe 100, a user will first need to remove the rigid needle shield 103. To do this the user may simply pull on the blinding cap 1. The blinding cap 1 pulls off the syringe 100 and carries with it the rigid needle shield 103 due to engagement of the inwardly-projecting flange 9 behind the proximal face 104 of the rigid needle shield 103. Thus, the needle 102 of the syringe 100 is exposed for injection. The rigid needle shield 103 stays retained within the blinding cap 1 even after removal of the blinding cap from the remainder of the syringe 100 and therefore remains effectively blinded.

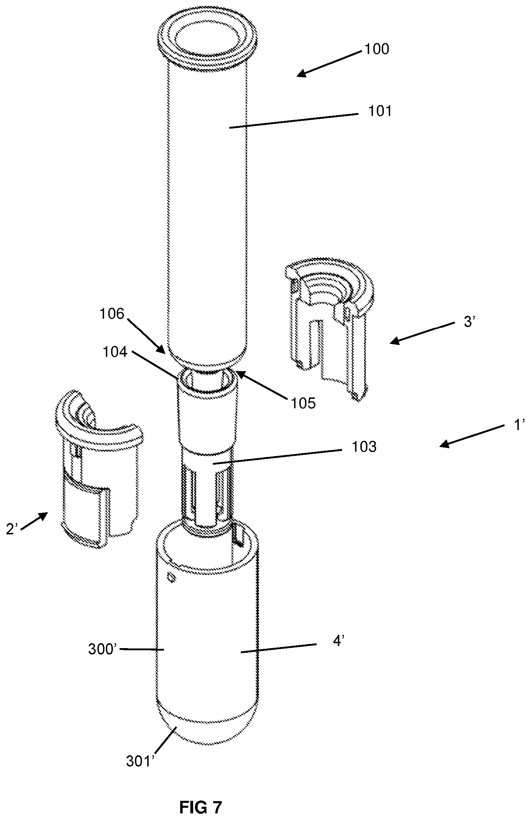

Figures 7 to 15 show a second blinding cap 1' useful for understanding the present disclosure. Unless otherwise stated, features of the second embodiment of blinding cap 1' are as described above with respect to the first embodiment of blinding cap 1 and reference should be made to the first embodiment for a full understanding of the second embodiment. The same or like features are referenced in the attached drawings using like reference numerals but with the addition of the prime suffix ( ' ). For reasons of brevity only, the following description will concentrate on features of the second embodiment which differ from those of the first embodiment.

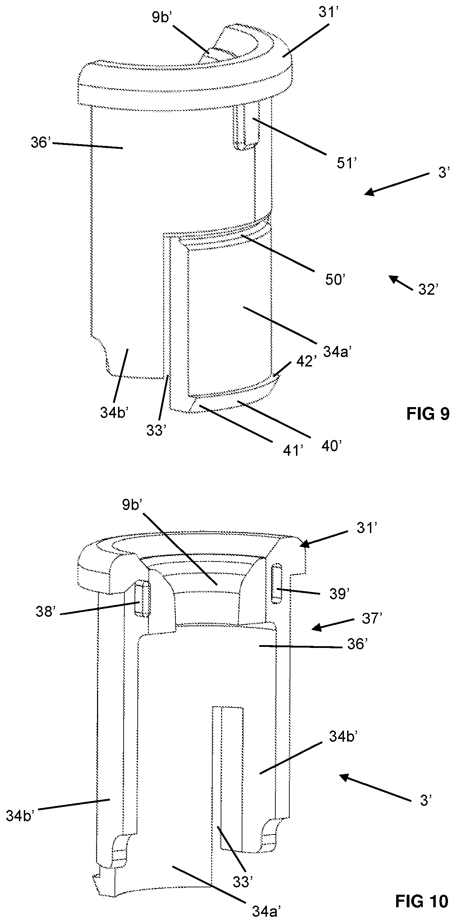

As best shown in Figure 10, the projection 28', 38' and the recess 29', 39' that are co-operatively shaped are each elongated in shape, for example so as to have a roughly rectangular shape, as opposed to being round as in the first embodiment. This may allow for a more secure temporary engagement of the first collar section 2' and the second collar section 3'.

Each of the first collar section 2' and the second collar section 3' may comprise only two longitudinal slits 23', 33'. Preferably the two longitudinal slits 23', 33' define for each collar section a central tongue 24a' and two outer tongues 24b', one on each side of the central tongue.

Figures 9 and 10 show the second collar section 3' in more detail. The first collar section 2' corresponds. Each central tongue 24a', 34a' may comprise a hinge portion 50' at its proximal end to permit flexing of the central tongue 24a', 34a' relative to the annular portion 26', 36' of the bodies 22', 32'. The hinge portion 50' may comprise a thinned section of material. The hinge portion 50' may comprise a living hinge.

Each of the outer tongues 24b', 34b' may be shorter than the central tongue 24a', 34a' such that the central tongue 24a', 34a' extends distally further than the outer tongues 24b', 34b'.

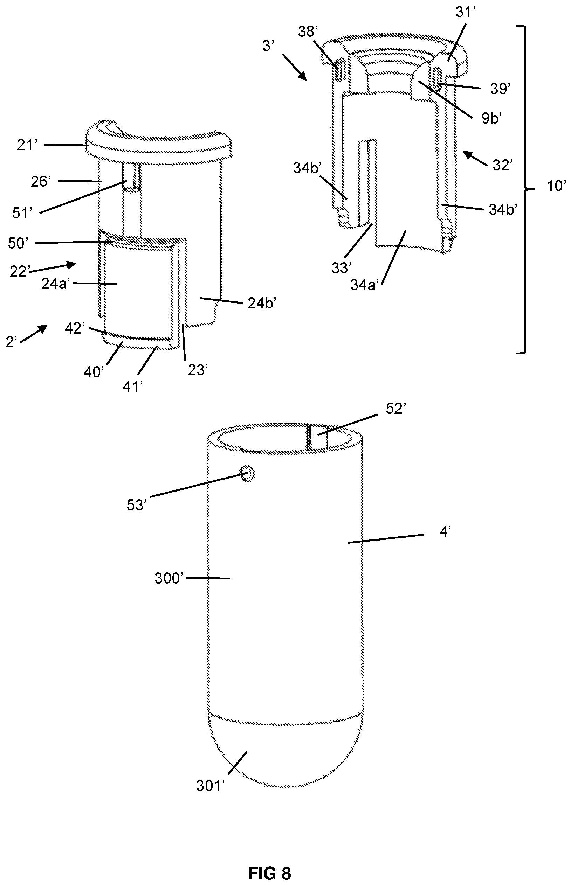

Each central tongue 24a', 34a' may be provided with an outwardly-projecting lip 40' at or near its distal end as shown in Figures 8 and 9. As in the first embodiment, the lip 40' may comprise a ramped surface 41' which is directed distally and a perpendicular face 42' located at a proximal end of the ramped surface 41'. The first part of the securement may comprise or consist of the lips 40' and the associated central tongues 24a', 34a'.

The outer tongues 24a', 34a' preferably are not provided with any lip at their distal ends.

One or both of the first collar section 2' and the second collar section 3' may also comprise a rotational locator 51'. As shown in Figures 8 and 9, the rotational locator 51' may comprise a projection provided on an outer surface of the bodies 22', 32'. The rotational locator may be provided on the outer surface of the annular portion 26', 36', preferably adjacent the head 21', 31'. The rotational locator 51' may be aligned with the central tongue 24a', 34a' of the respective collar section 2', 3'. A single rotational locator 51' may be provided on each collar section 2', 3'.

In this embodiment the inwardly-projecting flange 9' may, as in the first embodiment, be formed from a first portion 9a' on the first collar section 2' and a second portion 9b' on the second collar section 3'. As above the first collar section 2' and the second collar section 3' may each be formed from a single part, for example a single moulded part. In this embodiment the first portion 9a' and the second portion 9b' may extend along the whole or a majority of the bore 14' of the head section 11'. A proximal face of the first portion 9a' and the second portion 9b' may be rounded. The flange 9' formed by the coming together of the first portion 9a' and the second portion 9b' may be shaped and sized, in particular the rounded proximal face thereof, to interface with the shape and dimensions of the syringe 100, in particular the distal end 106 of the barrel 101.

As in the first embodiment, the first collar section 2' and the second collar section 3' may be identical to each other .

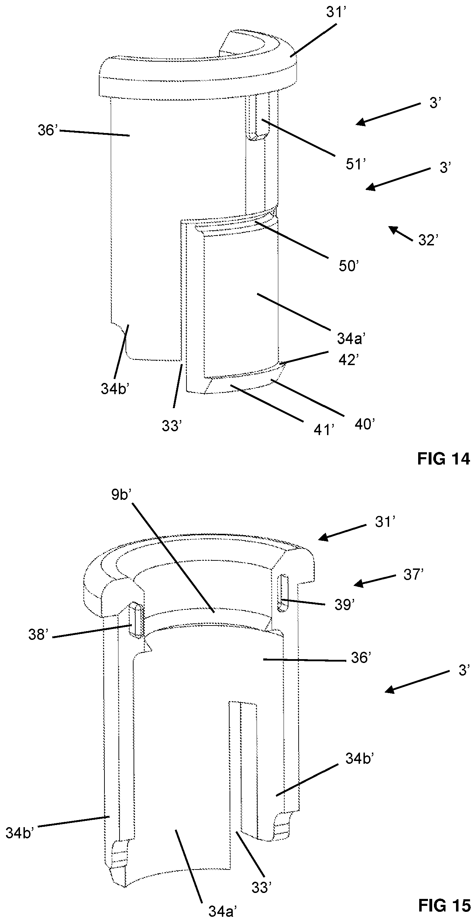

A variant version of the second collar section 3' is shown in Figures 14 and 15. Again, a variant version of the first collar section 2' may correspond. The design and configuration of the collar sections 2', 3' is the same as above, and shown in Figures 9 and 10, except that the first portion 9a' and the second portion 9b' of the inwardly-projecting flange 9' comprise a narrower lip. The lip may have a generally triangular cross-section and may be located at or adjacent the distal end of the head section 11' bordering the body section 12'. Again, the flange 9' formed by the coming together of the first portion 9a' and the second portion 9b' may be shaped and sized, in particular the rounded proximal face thereof, to interface with the shape and dimensions of the syringe 100, in particular the distal end 106 of the barrel 101.

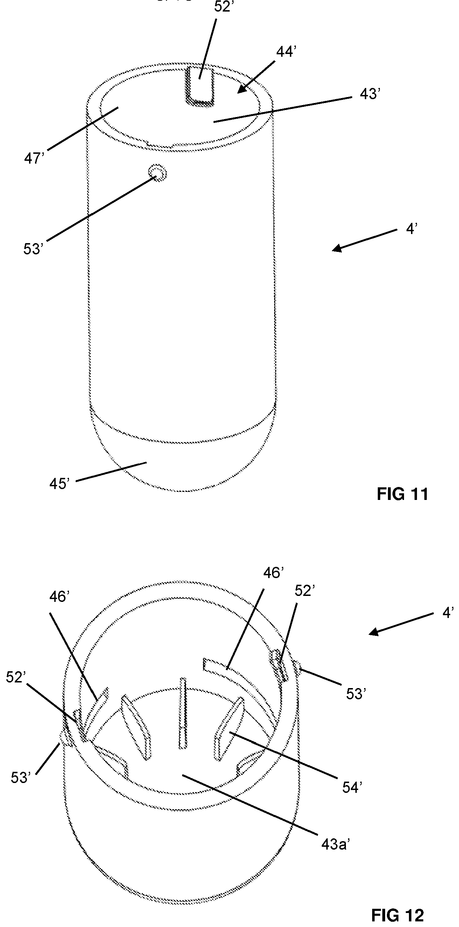

As best shown in Figures 11 to 13, the sleeve section 4' may differ to the first embodiment by the provision of two recesses 52' located adjacent a rim of the sleeve section 4'. The recesses 52' may be located diametrically opposite each other. The recesses 52' may be sized and shaped to receive the rotational locators 51' of the collar sections 2', 3'.

The sleeve section 4' may also be provided with one or two identifiers 53' of its outer face. The one or two identifiers 53' function to allow the recesses 52' to be aligned more easily with the rotational locators 51' when inserting the collar unit 10' into the sleeve section 4'. The identifier(s) 53' may comprise a pip or projection on the outer face of the sleeve section 4'.

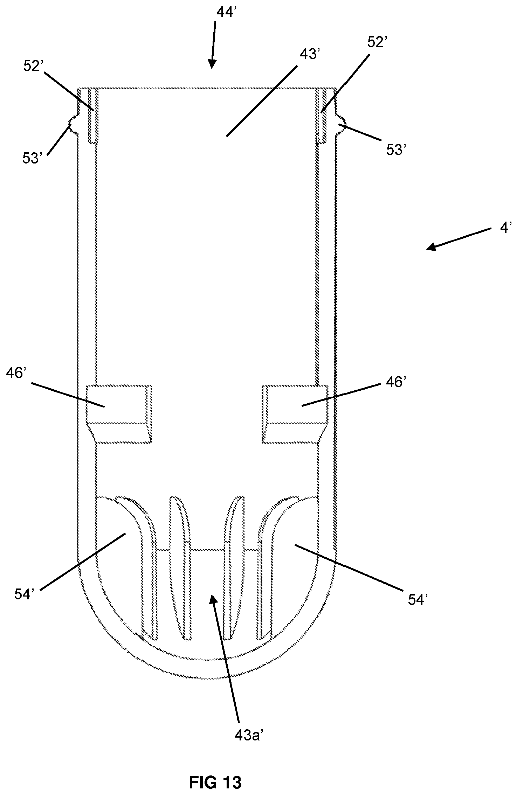

As shown in Figure 12 and 13, the sleeve section 4' may comprise two recesses 46' for receiving the outwardly-projecting lips 40' of the central tongues 24a', 34a'. Each recess 46' may be a part-annular recess which may extend around a portion of the inner face of the sleeve section 4'. Each recess 46' may have a circumferential length that is the same or marginally greater than the arcuate length of the lip 41' of each collar section 2', 3'. The second part of the securement may comprise or consist of the two recesses 46'.

The sleeve section 4' may further comprises a plurality of fins 54' at the base of the bore 43' within the closed second end 45'. The fins 54' may each extend radially from the wall of the sleeve section 4' towards, but stop short of, a centre of the bore 43'. The plurality of fins 54' may define a central narrower bore 43a' for receiving the rigid needle shield 103.

In use, as with the first embodiment, to assemble the blinding cap 1' and form the syringe assembly 200', the first collar section 2' and the second collar section 3' are brought together about the syringe 100. In this step the rigid needle shield 103 is accommodated in the body section 12' of the collar unit 10' in between the bodies 22', 32'. The inwardly-projecting flange 9' of the collar unit 10' is formed by the coming together of the first flange portion 9a' and the second flange portion 9b' so as to be located in the axial gap 105 between the end surface 104 of the rigid needle shield 103 and the distal end 106 of the barrel 101. The one or more projections 28', 38' are received in the one or more recesses 29', 39' to aid temporary alignment and retention of the first collar section 2' and the second collar section 3' on and about the rigid needle shield 103. The distal end 106 of the barrel 101 is received in the bore 14' of the head section 11'.

Next, the body section 12' of the collar unit 10' may be inserted into the sleeve section 4'. In this step the body section 12' passes through the open first end 44' into the bore 43'. The distal end 17' of the collar unit 10' may be configured to deform to accommodate insertion of the collar unit 10' into the sleeve section 4'. For example, the central tongues 24a', 34a' may flex inwardly about their hinge portions 50' due to engagement of the ramped surfaces 41' of the lips 40' engaging on a rim of the open first end 44'. Advantageously the presence of the hinge portions 50' may increase the flexibility of the central tongues 24a', 34a' allowing a lower insertion force to be required to assemble the blinding cap 1'.

The collar unit 10' (and syringe 100') are slid into the bore 43' until the two lips 40' reach the two recesses 46' at which point the central tongues 24a', 34a' resiliently flex back outwardly so as to engage the lips 40' in the recesses 46'. Preferably, at the same point of insertion the head section 11' is brought into contact against the proximal end of the sleeve section 4' without entering the bore 43' of the sleeve section 4'.

Alignment of the collar unit 10' with the sleeve section 4' may be facilitated by the user needing to rotationally align the rotational locators 51' on the collar unit 10' with the recesses 52' in the sleeve section 4'. This may ensure that the lips 40' are aligned with the recesses 46' during the final stage of the insertion.

Advantageously the engagement of the two lips 40' in the two recesses 46' may help to prevent rotation or twisting of the collar unit 10' within the sleeve section 4'. Additionally or alternatively the engagement of the rotational locators 51' in the recesses 52' may help to prevent rotation or twisting of the collar unit 10' within the sleeve section 4. Preventing twisting or rotation of the collar unit 10' may help to increase the integrity of the securement of the collar unit 10' within the sleeve section 4'.

On insertion the rigid needle shield 103 may be located in the central narrower bore 43a' between the plurality of fins 54'. The fins 54' may function to reduce lateral movement of the rigid needle shield 103 within the blinding cap 1'. Preferably the fins 54' grip the rigid needle shield 103 so as to limit or prevent rattling of the rigid needle shield within the blinding cap 1'.

Removal of the collar unit 10' from the sleeve section 4' is resisted by engagement of the perpendicular face 42' of the lips 40' against a proximal edge of the annular recesses 46'. Thus, as in the first embodiment the securement acts as a non-reversible means for connecting the collar unit 10' and the sleeve section 4'.

Removal of the blinding cap 1' to use the syringe 100 is as described above for the first embodiment.

The blinding cap 1, 1', the syringe assembly 200, 200' and the method of the present disclosure finds particular application in the field of clinical trials, especially blinded trials. For example, the present disclosure extends to a method of assembling a syringe assembly 200, 200' from a plurality of separate components that comprise a first collar section 2, 2', a second collar section 3, 3', a sleeve section 4, 4' and a syringe 100 that itself comprises a barrel 101 and a rigid needle shield 103. The method comprises first encircling the rigid needle shield 103 of the syringe 100 by engaging the first collar section 2, 2'and the second collar section 3, 3' together to form a collar unit 10, 10' that encircles the rigid needle shield 103. The collar unit 10, 10' comprises an inwardly-projecting flange 9, 9' that is disposed between an end surface of the rigid needle shield 103 and the barrel 101 of the syringe 100. Subsequently the collar unit 10, 10' and the encircled rigid needle shield 103 is inserted into the sleeve section 4, 4'. Insertion of the collar unit 10, 10' into the sleeve section 4, 4' results in a first part of a securement, provided on the collar unit 10, 10', and a second part of the securement, provided on the sleeve section 4, 4', securely engaging one another to securely retain the collar unit 10, 10' within the sleeve section 4, 4'.

Advantageously, the blinding cap of the present disclosure may provide greater security in that it is generally more difficult to tamper with or separate the first collar section and second collar section from each other due to the presence of the sleeve section. Thus, the first collar section and second collar section cannot readily be opened once the blinding cap has been assembled.

In addition, the blinding cap may be easier to assemble onto the syringe than prior art devices. In particular, the first collar section and the second collar section may be aligned with each other to encircle the rigid needle shield in a straightforward manner. The securing of the collar sections together may be achieved subsequently by their insertion into the sleeve section. Therefore, the steps of aligning the collar sections and securing the collar sections together are achieved sequentially which may lead to a simpler method of assembly.

In addition, the sleeve section beneficially serves the dual functions of securing the first collar section and the second collar section together and of blinding at least a portion of the rigid needle shield, for example a distal portion of the rigid needle shield.

Advantageously, the combination of the sleeve section and the collar unit may ensure full blinding of the rigid needle shield since line-of-sight through the proximal opening will, in use, be prevented by the presence of the barrel of the syringe which will abut or be in close proximity with the inwardly-projecting flange.

Advantageously, the alignment locators may ease the temporary relative alignment of the first collar section and the second collar section until they can be inserted into the sleeve section.

Advantageously, tampering with the securement is made more difficult by arranging the first part of the securement fully within the sleeve section once assembly of the blinding cap has been completed. In addition, the second part of the securement may be provided on an internal surface of the sleeve section, for example on an internal surface of the bore of the sleeve section. Thus, advantageously, neither part of the securement may be accessible from an exterior of the blinding cap once assembled.

Advantageously, in this manner a very secure assembly of the blinding cap may be achieved since the collar unit is engaged with the sleeve section around the full or substantially the full circumference of the blinding cap, rather than being secured simply along one or two longitudinal seams.

Advantageously, the head section may function to receive and obscure from sight an end of the syringe barrel.

Advantageously, by configuring the blinding cap to have a smooth exterior surface the syringe assembly may be easier to package, in particular in a flow-wrap package.

Advantageously, the plurality of fins may act to grip the rigid needle shield to limit or prevent rattling or movement of the rigid needle shield within the blinding cap.

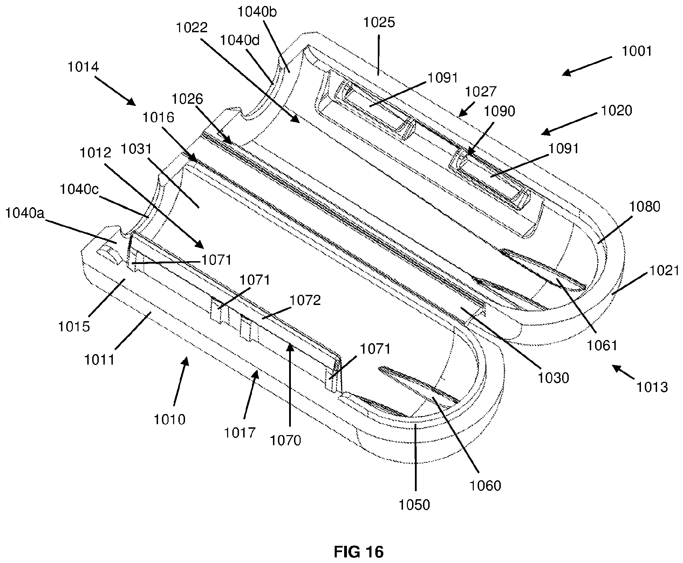

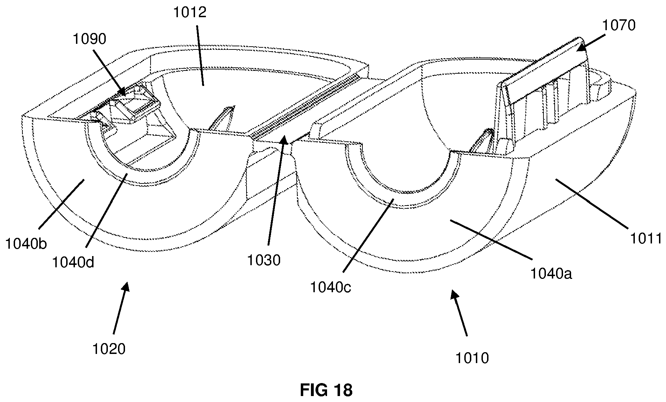

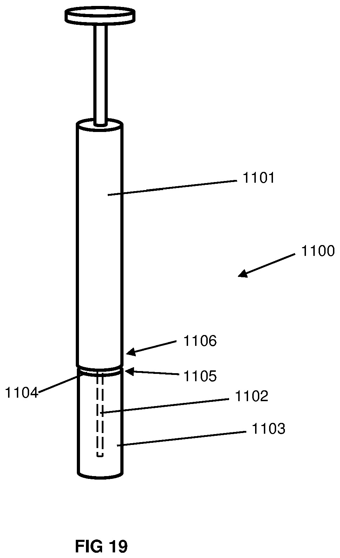

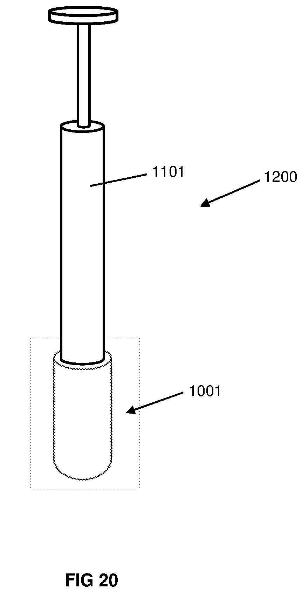

Figures 16 to 18 show a third embodiment of blinding cap 1001 according to the present disclosure. The blinding cap 1001 is intended to be combined with a syringe 1100, shown schematically in Figure 19, to form a syringe assembly 1200, as shown schematically in Figure 20. The blinding cap 1001 functions to cover a rigid needle shield 1103 that is releasably mounted on a barrel 1101 of the syringe 1100 to shield a needle 1102 of the syringe 1100 as shown in Figure 19.

The blinding cap 1001 comprises a first part 1010 and a second part 1020 that are joined together by a hinge 1030.

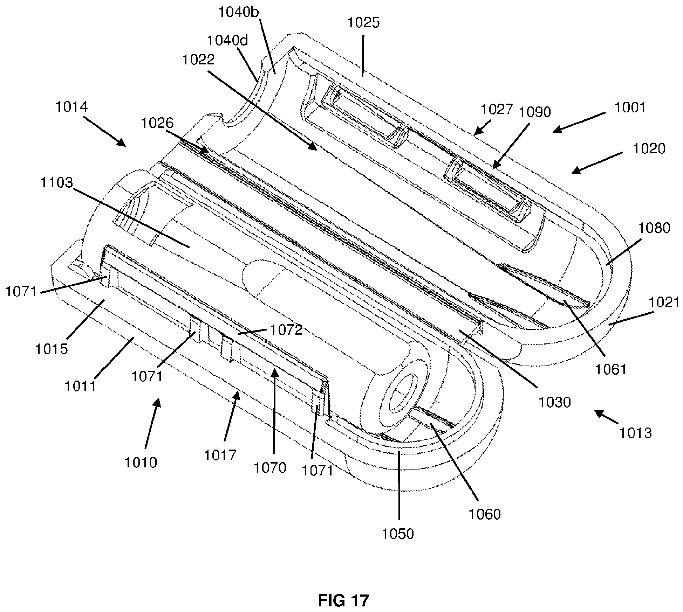

The first part 1010 comprises a first elongate bowl-shaped body 1011 defining a first recess 1012. The recess 1012 (together with a second recess 1022 described below) may be shaped and sized to accommodate the rigid needle shield 1103 as shown in Figure 17. The first elongate bowl-shaped body 1011 may have a first lateral side 1016 and a second lateral side 1017 that each extend between a distal end 1013 and a proximal end 1014. The first part 1010 may be generally hollow. The first part 1010 may be generally thin-walled. The first part 1010 may comprise a first half shell.

A distal end 1013 of the first part may be rounded.

A first flange portion 1040a is located at the proximal end 1014. The first flange portion 1040a may define a first portion of a central aperture of the proximal end 1014. A distal edge of the first flange portion 1040a defining the first portion of the central aperture may comprise a chamfer 1040c.

A first rim 1015 extends at least partially around an open mouth of the first elongate bowl-shaped body 1011. A raised lip 1050 extends at least partially along the first rim 1015. The raised lip 1050 may extend around the rounded distal end 1013 of the first part 1010. The raised lip 1050 may extend along the lateral side 1016 of the first elongate bowl-shaped body proximate the hinge 1030.

One or more fins 1060 project inwardly from the first elongate bowl-shaped body 1011. The one or more fins 1060 may be flexible or rigid. The one or more fins 1060 may comprise a free edge that is curved and/or tapered so as to define an engagement surface for a side face and or corner of the rigid needle shield 1103.

A first locking member 1070 is provided. The first locking member 1070 may comprise a locking frame comprises a plurality of struts 1071 extending from the first rim 1015 interconnected by one or more locking bars 1072. The first locking member 1070 may be provided on the lateral side 1017 of the first elongate bowl-shaped body 1011 opposite the hinge 1030. The first locking member 1070 may project above the first rim 1015.

The first part 1010 may be opaque.

The second part 1020 comprises a second elongate bowl-shaped body 1021 defining a second recess 1022. The second elongate bowl-shaped body 1021 may have a first lateral side 1026 and a second lateral side 1027 that each extend between the distal end 1013 and the proximal end 1014. The second part 1020 may be generally hollow. The second part 1020 may be generally thin-walled. The second part 1020 may comprise a second half shell.

The distal end 1013 of the second part 1020 may be rounded.

A second flange portion 1040b is located at the proximal end 1014. The second flange portion 1040b may define a second portion of the central aperture of the proximal end 1014. A distal edge of the second flange portion 1040b defining the second portion of the central aperture may comprise a chamfer 1040d.

A second rim 1025 extends at least partially around an open mouth of the second elongate bowl-shaped body 1021. A recess 1080 extends at least partially along the second rim 1025. The recess 1080 may extend around the rounded distal end 1013 of the second part 1020.

One or more fins 1061 project inwardly from the second elongate bowl-shaped body 1021. The one or more fins 1061 may be flexible or rigid. The one or more fins 1061 may comprise a free edge that is curved and/or tapered so as to define an engagement surface for a side face and or corner of the rigid needle shield 1103.

A second locking member 1090 is provided. The second locking member 1090 may comprise one or more locking tabs 1091 configured to interengage with the one or more locking bars 1072 by flexure of the struts 1071 and/or the locking tabs 1091. The second locking member 1090 may be recessed below the second rim 1025.

The second part 1090 may be opaque.

The hinge 1030 may be an elongate hinge. The hinge 1030 may extend over greater than 50%, optionally 70%, optionally 90% of the length of the first lateral side 1016 of the first elongate bowl-shaped body 1011 and/or the first lateral side 1026 of the second elongate bowl-shaped body 1021 that may be proximate the hinge 1030.

The blinding cap 1001 may comprise a single unitary part. Alternatively, each of the first part 1010 and the second part 1020 may be single unitary parts. The hinge 1030 may be formed unitarily with the first part 1010 and/or the second part 1020. Alternatively, the hinge 1030 may be separately formed and joined to the first part 1010 and the second part 1020.

The first part 1010 and/or the second part 1020 and/or the blinding cap 1001 as a whole may be a moulded part and may optionally be formed of a rigid plastics material.

The blinding cap 1001 may have a smooth exterior surface. For example, the blinding cap 1001 may comprise a cylindrical side wall and a rounded distal end so that it is free of abrupt discontinuities.

In use, the blinding cap 1001 is configured such that the first part 1010 and the second part 1020 can be brought into engagement around the rigid needle shield 1103 by flexure of the hinge 1030. Typically, the rigid needle shield 1103 is already coupled to the syringe 1100 before the blinding cap 1001 is engaged around the rigid needle shield. During engagement of the first part 1010 and the second part 1020, the raised lip 1050 of the first part 1010 is engaged in the recess 1080 of the second part 1020. Additionally, the first locking member 1070 and the second locking member 1090 engage one another to lock the first part 1010 and the second part 1020 together. Additionally, the first recess 1012 and second recess 1022 together define a cavity for receiving the rigid needle shield 1103.

Further, the first flange portion 1040a and the second flange portion 1040b are juxtaposed to form an inwardly-projecting flange that is configured to be disposed between the end surface 1104 of the rigid needle shield 1103 and a barrel of the syringe. The inwardly-projecting flange may define the central aperture through which a portion of the syringe may project. The inwardly-projecting flange may comprise a chamfered edge constituted by the chamfers 1040c and 1040d of the first flange portion 1040a and the second flange portion 1040b. The inwardly-projecting flange may be located in an axial gap 1105 between the end surface 1104 of the rigid needle shield 1103 and a distal end 1106 of the barrel 1101. The chamfer of the inwardly-projecting flange may beneficially ease the fitting of the blinding cap 1001 onto the syringe 1100. It may also allow for a closer fitting of the blinding cap 1001 to the small diameter section of the syringe 1100 and additionally may act as a means to accommodate slight variations in the diameter of the syringe 1100.

The one or more fins 1060 of the first part 1010 and the one or more fins 1061 of the second part 1020 are configured to engage the rigid needle shield 1103 on closure of the blinding cap 1001. The one or more fins 1060 of the first part 1010 and the one or more fins 1061 of the second part 1020 may together define a narrower bore within the cavity for engaging the rigid needle shield 1103.

The blinding cap 1001, the syringe 1100 and the rigid needle shield 1103 may together comprise a syringe assembly 1200. The syringe 1200 may be a manually-activated syringe.

The syringe assembly 1200 may be stored in packaging, for example a flow wrap packaging.

To use the syringe 1100, a user will first need to remove the rigid needle shield 1103. To do this the user may simply pull on the blinding cap 1001. The blinding cap 1001 pulls off the syringe 1100 and carries with it the rigid needle shield 1103 due to engagement of the inwardly-projecting flange behind the proximal face 1104 of the rigid needle shield 1103. Thus, the needle 1102 of the syringe 1100 is exposed for injection. The rigid needle shield 1103 stays retained within the blinding cap 1001 even after removal of the blinding cap from the remainder of the syringe 1100 and therefore remains effectively blinded.

Fourth, fifth and sixth embodiments of the disclosure will now be described. Unless otherwise stated, features of these embodiments of blinding cap are as described above with respect to the third embodiment of blinding cap 1001 and reference should be made to the third embodiment for a full understanding of these embodiments. In particular, the fourth, fifth and sixth embodiments exhibit differences in the design of the one or more fins for engaging the rigid needle shield. For reasons of brevity only, the following description will concentrate on features of these embodiments which differ from those of the third embodiment.

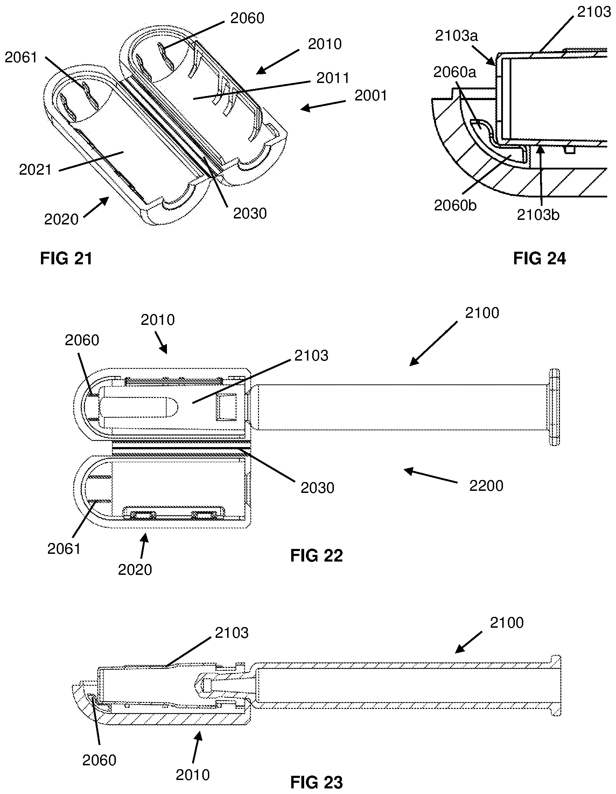

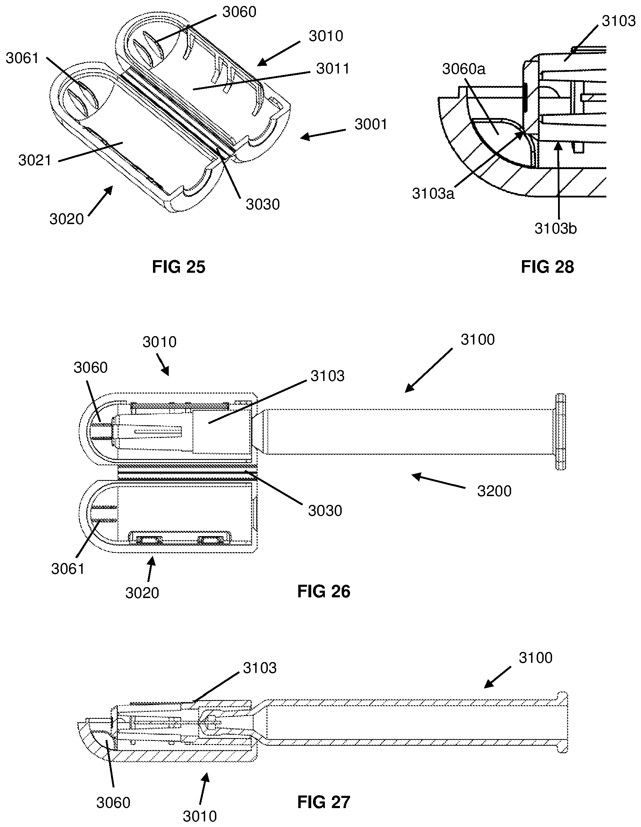

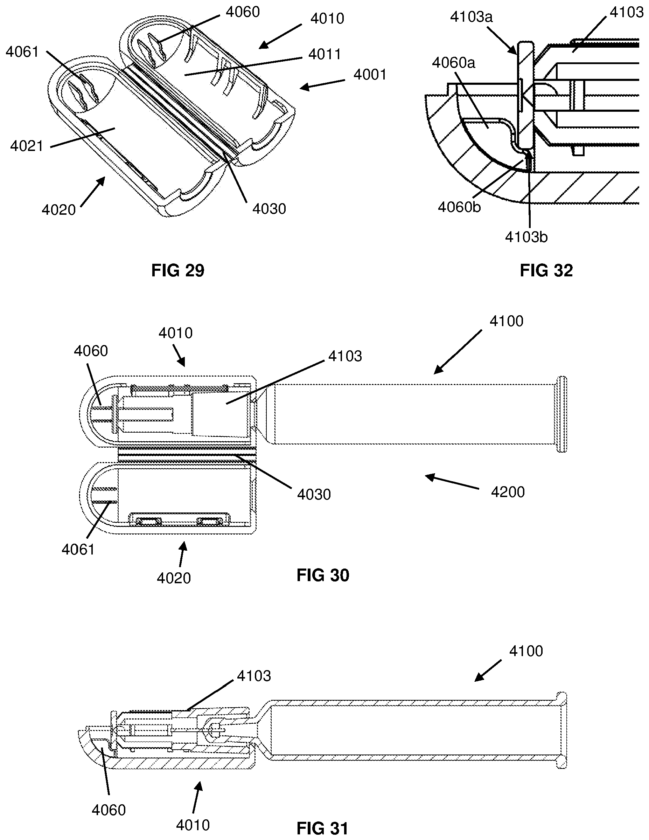

The fourth embodiment is shown in Figures 21 to 24. The fifth embodiment is shown in Figures 25 to 28. The sixth embodiment is shown in Figures 29 to 32.

In the fourth, fifth and sixth embodiments one or more fins 2060, 3060, 4060 project inwardly from the first elongate bowl-shaped body 2011, 3011, 4011 of the first part 2010, 3010, 4010. Two fins 2060, 3060, 4060 may be provided. The fins 2060, 3060, 4060 may be parallel to one another and parallel to the longitudinal axis of the first part 2010, 3010, 4010. In addition, one or more fins 2061, 3061, 4061 project inwardly from the second elongate bowl-shaped body 2021, 3021, 4021 of the second part 2020, 3020, 4020. Two fins 2061, 3061, 4061 may be provided. The fins 2061, 3061, 4061 may be parallel to one another and parallel to the longitudinal axis of the second part 2020, 3020, 4020.

The fins 2060, 3060, 4060, 2061, 3061, 4061 may be flexible and may optionally have a thickness of approximately 0.15 mm. However, preferably the fins 2060, 3060, 4060, 2061, 3061, 4061 may be rigid. They may be moulded from a rigid engineering plastic. They may be integrally moulded as part of the respective first part 2010, 3010, 4010 or second part 2020, 3020, 4020. The fins 2060, 3060, 4060, 2061, 3061, 4061 may have a thickness of approximately 0.4 mm.

In the fourth embodiment, as most clearly shown in Figure 24, the fins 2061 may comprise a generally L-shaped configuration. (The fins 2062 may be configured in the same way). Each fin 2061 may comprise a first portion 2060a and a second portion 2060b. The first portion 2060a may define an end stop for an end face 2103a of the rigid needle shield 2103. In so doing the end stop may limit longitudinal movement of the rigid needle shield 2103 within the blinding cap 2001. The second portion 2060b may define a side stop for a side face 2103b of the rigid needle shield 2103. In so doing the side stop may limit transverse or sideways movement of the rigid needle shield 2103 within the blinding cap 2001. The second portion 2060b may also define a narrower bore within the cavity for engaging and/or receiving a portion of the rigid needle shield 2103. The fins 2061, 2062 may provide a very close fit for the rigid needle shield 2103 with a resultant significant reduction in the amount of rattling of the rigid needle shield 2103 within the blinding cap 2001.

In the fifth embodiment, as most clearly shown in Figure 28, the fins 3061 may comprise a generally arcuate configuration. (The fins 3062 may be configured in the same way). Each fin 3061 may comprise a first portion 3060a that has a rounded free edge. The first portion 3060a may define a corner stop for a corner 3103a of the rigid needle shield 3103. In so doing the corner stop may limit longitudinal and transverse or sideways movement of the rigid needle shield 3103 within the blinding cap 3001. In this way a very close fit for the rigid needle shield 3103 may be provided with a resultant significant reduction in the amount of rattling of the rigid needle shield 3103 within the blinding cap 3001.

In the sixth embodiment, as most clearly shown in Figure 32, the configuration of the fins 4060 is similar to that of the fourth embodiment. However, in this embodiment the length of the second portion 4060b (in the longitudinal direction) is reduced compared to the fourth embodiment. The end stop and side stop of the fins 4060 function in the same way however.

Advantageously, as shown by way of example in the fourth, fifth and sixth embodiments, the size and shape of the fins may be configured to engage a range of sizes and shapes of rigid needle shield without altering the external appearance of the blinding cap.

The present disclosure also extends to a method of preparing materials for carrying out a clinical trial, preferably a blinded clinical trial, which comprises the method of assembling the syringe assembly 200, 200', 1200, 2200, 3200, 4200 described above. In addition, the method of preparing materials for carrying out the clinical trial may further comprise assembling a plurality of said syringe assemblies 200, 200', 1200, 2200, 3200, 4200 according to said method, wherein a first group of the plurality of syringe assemblies 200, 200', 1200, 2200, 3200, 4200 share a first characteristic and a second group of the plurality of syringe assemblies 200, 200', 1200, 2200, 3200, 4200 share a second, different characteristic. The characteristic may be, by way of example, one or more of: a type of medicament, a dosage of medicament, and the presence or absence of an active medicament - i.e. the first group may comprise an active medicament and the second group may comprise a placebo.

It is to be understood that at least some of the figures and descriptions of the disclosure have been simplified to focus on elements that are relevant for a clear understanding of the disclosure, while eliminating, for purposes of clarity, other elements that the reader skilled in the art will appreciate may also be required. Because such elements are well known to the reader skilled in the art, and because they do not necessarily facilitate a better understanding of the disclosure, a description of such elements is not provided herein.

## Claims

1. A blinding cap (1001, 2001, 3001, 4001) for covering a rigid needle shield (1103, 2103, 3103, 4103) that is releasably mounted on a syringe (1100), the blinding cap (1001, 2001, 3001, 4001) comprising a first part (1010, 2010, 3010, 4010) and a second part (1020, 2020, 3020, 4020) joined together by a hinge (1030, 2030, 3030, 4030);
the first part (1010, 2010, 3010, 4010) comprising:
- a first elongate bowl-shaped body (1011, 2011, 3011, 4011) defining a first recess (1012);
- a first flange portion (1040a) located at a proximal end (1014);
- a first rim (1015) extending at least partially around an open mouth of the first elongate bowl-shaped body (1011, 2011, 3011, 4011);
- a raised lip (1050) extending at least partially along the first rim (1015);
- one or more fins (1060, 2060, 3060, 4060) that project inwardly from the first elongate bowl-shaped body (1011, 2011, 3011, 4011); and
- a first locking member (1070);
the second part (1020, 2020, 3020, 4020) comprising:
- a second elongate bowl-shaped body (1021, 2021, 3021, 4021) defining a second recess (1022);
- a second flange portion (1040b) located at a proximal end (1014);
- a second rim (1025) extending at least partially around an open mouth of the second elongate bowl-shaped body (1021, 2021, 3021, 4021);
- a recess (1080) extending at least partially along the second rim (1025);
- one or more fins (1061, 2061, 3061, 4061) that project inwardly from the second elongate bowl-shaped body (1021, 2021, 3021, 4021); and
- a second locking member (1090);
wherein the blinding cap (1001, 2001, 3001, 4001) is configured such that the first part (1010, 2010, 3010, 4010) and the second part (1020, 2020, 3020, 4020) can be brought into engagement around the rigid needle shield (1103, 2103, 3103, 4103) by flexure of the hinge (1030, 2030, 3030, 4030) such that:
i) the raised lip (1050) of the first part (1010, 2010, 3010, 4010) is engaged in the recess (1080) of the second part (1020, 2020, 3020, 4020);
ii) the first locking member (1070) and the second locking (1090) member engage one another to lock the first part (1010, 2010, 3010, 4010) and the second part (1020, 2020, 3020, 4020) together;
iii) the first recess (1012) and second recess (1022) together define a cavity for receiving the rigid needle shield (1103, 2103, 3103, 4103) of the syringe (1100, 2100, 3100, 4100);
iv) the first flange portion (1040a) and the second flange portion (1040b) are juxtaposed to form an inwardly-projecting flange that is configured to be disposed between an end surface (1104) of the rigid needle shield (1103, 2103, 3103, 4103) and a barrel of the syringe (1100, 2100, 3100, 4100); and
v) the one or more fins (1060, 2060, 3060, 4060, 1061, 2061, 3061, 4061) of the first part (1010, 2010, 3010, 4010) and the second part (1020, 2020, 3020, 4020) are configured to engage the rigid needle shield (1103, 2103, 3103, 4103).

2. The blinding cap (1001) of claim 1, wherein the one or more fins (1060, 1061) of the first part (1010) and the second part (1020) together define a narrower bore within the cavity for engaging the rigid needle shield (1103).

3. The blinding cap (1001, 2001, 3001, 4001) of claim 1 or claim 2, wherein the one or more fins (2060, 4060, 2061, 4061) of the first part (2010, 4010) and the second part (2020, 4020) comprise a first portion (2060a, 4060a) defining an end stop for an end face (2103a, 4103a) of the rigid needle shield (2103) and/or a second portion (2060b, 4060b) defining a side stop for a side face (2103b, 4103b) of the rigid needle shield (2103, 4103) and/or a corner portion defining a corner stop for a corner (3103a) of the rigid needle shield (1103, 3103).

4. The blinding cap (1001, 2001, 3001, 4001) of any preceding claim, wherein the one or more fins (1060, 2060, 3060, 4060, 1061, 2061, 3061, 4061) of the first part (1010, 2010, 3010, 4010) and the second part (1020, 2020, 3020, 4020) comprise one or more flexible fins or one or more rigid fins.

5. The blinding cap (1001, 2001, 3001, 4001) of any preceding claim, wherein the inwardly-projecting flange defines a proximal opening at a proximal end (1014).

6. The blinding cap (1001, 2001, 3001, 4001) of any preceding claim, wherein the inwardly-projecting flange comprises a chamfered edge.

7. The blinding cap (1001, 2001, 3001, 4001) of any preceding claim, wherein a distal end (1013) of the first part (1010) and the second part (1020) is rounded such that on engagement of the first part (1010) and the second part (1020) the blinding cap (1001, 2001, 3001, 4001)comprises a closed, rounded distal end (1013).

8. The blinding cap (1001, 2001, 3001, 4001) of claim 7, wherein the raised lip (1050) extends around the rounded distal end (1013) of the first part (1010) and the recess (1080) extends around the rounded distal end (1013) of the second part (1020).

9. The blinding cap (1001, 2001, 3001, 4001) of any preceding claim, wherein the raised lip (1050) extends along a lateral side (1016) of the first elongate bowl-shaped body (1011) proximate the hinge (1030).

10. The blinding cap (1001, 2001, 3001, 4001) of any preceding claim, wherein the first locking member (1070) is provided on a lateral side (1017) of the first elongate bowl-shaped body (1011) opposite the hinge (1030).

11. The blinding cap (1001, 2001, 3001, 4001) of any preceding claim, wherein one of the first (1070) and second (1090) locking members projects above the first rim (1015) and the other of the first (1070) and second (1090) locking members
is recessed below the second rim (1025).

12. The blinding cap (1001, 2001, 3001, 4001) of any preceding claim, wherein:
the first locking member (1070) comprises a locking frame comprises a plurality of struts (1071) extending from the first rim (1015) interconnected by one or more locking bars (1072); and
the second locking member (1090) comprises one or more locking tabs (1091) configured to interengage with the one or more locking bars (1072) by flexure of the struts (1071).

13. The blinding cap (1001, 2001, 3001, 4001) of any preceding claim, wherein the first part (1010, 2010, 3010, 4010) and the second part (1020, 2020, 3020, 4020) are opaque.

14. A syringe assembly (1200, 2200, 3200, 4200) comprising a blinding cap (1001, 2001, 3001, 4001) according to any preceding claim and a syringe (1100) that comprises a barrel (1101) and a rigid needle shield (1103, 2103, 3103, 4103) that is releasably mounted over a needle of the syringe (1100);
and optionally, wherein the syringe (1100) is a manually-activated syringe.

## Patentansprüche

1. Verblindungskappe (1001, 2001, 3001, 4001) zum Abdecken einer starren Nadelabschirmung (1103, 2103, 3103, 4103), die lösbar an einer Spritze (1100) angebracht ist, wobei die Verblindungskappe (1001, 2001, 3001, 4001) einen ersten Teil (1010, 2010, 3010, 4010) und einem zweiten Teil (1020, 2020, 3020, 4020) umfasst, die durch ein Scharnier (1030, 2030, 3030, 4030) miteinander verbunden sind;
wobei der erste Teil (1010, 2010, 3010, 4010) umfasst:
- einen ersten langgestreckten schalenförmigen Körper (1011, 2011, 3011, 4011), der eine erste Aussparung (1012) definiert;
- einen an einem proximalen Ende (1014) angeordneten ersten Flanschabschnitt (1040a);
- einen ersten Rand (1015), der sich zumindest teilweise um eine offene Mündung des ersten langgestreckten schalenförmigen Körpers (1011, 2011, 3011, 4011) erstreckt;
- eine erhöhte Lippe (1050), die sich zumindest teilweise entlang des ersten Randes (1015) erstreckt;
- eine oder mehrere Lamellen (1060, 2060, 3060, 4060), die von dem ersten langgestreckten schalenförmigen Körper (1011, 2011, 3011, 4011) nach innen vorstehen; und
- ein erstes Verriegelungselement (1070);
wobei der zweite Teil (1020, 2020, 3020, 4020) umfasst:
- einen zweiten langgestreckten schalenförmigen Körper (1021, 2021, 3021, 4021), der eine zweite Aussparung (1022) definiert;
- einen an einem proximalen Ende (1014) angeordneten zweiten Flanschabschnitt (1040b);
- einen zweiten Rand (1025), der sich zumindest teilweise um eine offene Mündung des zweiten langgestreckten schalenförmigen Körpers (1021, 2021, 3021, 4021) erstreckt;
- eine Aussparung (1080), die sich zumindest teilweise entlang des zweiten Randes (1025) erstreckt;
- eine oder mehrere Lamellen (1061, 2061, 3061, 4061), die von dem zweiten langgestreckten schalenförmigen Körper (1021, 2021, 3021, 4021) nach innen vorstehen; und
- ein zweites Verriegelungselement (1090);
wobei die Verblindungskappe (1001, 2001, 3001, 4001) so konfiguriert ist, dass der erste Teil (1010, 2010, 3010, 4010) und der zweite Teil (1020, 2020, 3020, 4020) durch Biegen des Scharniers (1030, 2030, 3030, 4030) um die starre Nadelabschirmung (1103, 2103, 3103, 4103) herum so in Eingriff gebracht werden können, dass:
i) die erhöhte Lippe (1050) des ersten Teils (1010, 2010, 3010, 4010) in die Aussparung (1080) des zweiten Teils (1020, 2020, 3020, 4020) eingreift;
ii) das erste Verriegelungselement (1070) und das zweite Verriegelungselement (1090) ineinander greifen, um den ersten Teil (1010, 2010, 3010, 4010) und den zweiten Teil (1020, 2020, 3020, 4020) aneinander zu verriegeln;
iii) die erste Aussparung (1012) und die zweite Aussparung (1022) zusammen einen Hohlraum zur Aufnahme der starren Nadelabschirmung (1103, 2103, 3103, 4103) der Spritze (1100, 2100, 3100, 4100) bilden;
iv) der erste Flanschabschnitt (1040a) und der zweite Flanschabschnitt (1040b) nebeneinander angeordnet sind, um einen nach innen vorstehenden Flansch zu bilden, der konfiguriert ist, um zwischen einer Endfläche (1104) der starren Nadelabschirmung (1103, 2103, 3103, 4103) und einem Zylinder der Spritze (1100, 2100, 3100, 4100) angeordnet zu werden; und
v) die ein oder mehreren Lamellen (1060, 2060, 3060, 4060, 1061, 2061, 3061, 4061) des ersten Teils (1010, 2010, 3010, 4010) und des zweiten Teils (1020, 2020, 3020, 4020) konfiguriert sind, um mit der starren Nadelabschirmung (1103, 2103, 3103, 4103) in Eingriff zu kommen.

2. Verblindungskappe (1001) nach Anspruch 1, bei der die ein oder mehreren Lamellen (1060, 1061) des ersten Teils (1010) und des zweiten Teils (1020) zusammen eine schmalere Bohrung innerhalb des Hohlraums zum Eingriff mit der starren Nadelabschirmung (1103) definieren.

3. Verblindungskappe (1001, 2001, 3001, 4001) nach Anspruch 1 oder Anspruch 2, bei der die ein oder mehreren Lamellen (2060, 4060, 2061, 4061) des ersten Teils (2010, 4010) und des zweiten Teils (2020, 4020) einen ersten Abschnitt (2060a, 4060a), der einen Endanschlag für eine Endfläche (2103a, 4103a) der starren Nadelabschirmung (2103) definiert, und/oder einen zweiten Abschnitt (2060b, 4060b), der einen seitlichen Anschlag für eine Seitenfläche (2103b, 4103b) der starren Nadelabschirmung (2103, 4103) definiert, und/oder einen Eckabschnitt, der einen Eckanschlag für eine Ecke (3103a) der starren Nadelabschirmung (1103, 3103) definiert, umfassen.

4. Verblindungskappe (1001, 2001, 3001, 4001) nach einem der vorhergehenden Ansprüche, bei der die ein oder mehreren Lamellen (1060, 2060, 3060, 4060, 1061, 2061, 3061, 4061) des ersten Teils (1010, 2010, 3010, 4010) und des zweiten Teils (1020, 2020, 3020, 4020) eine oder mehrere flexible Lamellen oder eine oder mehrere starre Lamellen umfassen.

5. Verblindungskappe (1001, 2001, 3001, 4001) nach einem der vorhergehenden Ansprüche, bei der der nach innen vorstehende Flansch eine proximale Öffnung an einem proximalen Ende (1014) definiert.

6. Verblindungskappe (1001, 2001, 3001, 4001) nach einem der vorhergehenden Ansprüche, bei der der nach innen vorstehende Flansch eine abgeschrägte Kante umfasst.

7. Verblindungskappe (1001, 2001, 3001, 4001) nach einem der vorhergehenden Ansprüche, bei der ein distales Ende (1013) des ersten Teils (1010) und des zweiten Teils (1020) so abgerundet ist, dass beim Ineinandergreifen des ersten Teils (1010) und des zweiten Teils (1020) die Verblindungskappe (1001, 2001, 3001, 4001) ein geschlossenes, abgerundetes distales Ende (1013) umfasst.

8. Verblindungskappe (1001, 2001, 3001, 4001) nach Anspruch 7, bei der sich die erhöhte Lippe (1050) um das abgerundete distale Ende (1013) des ersten Teils (1010) erstreckt und die Aussparung (1080) sich um das abgerundete distale Ende (1013) des zweiten Teils (1020) erstreckt.

9. Verblindungskappe (1001, 2001, 3001, 4001) nach einem der vorhergehenden Ansprüche, bei der sich die erhöhte Lippe (1050) entlang einer zum Scharnier (1030) benachbarten seitlichen Flanke (1016) des ersten langgestreckten schalenförmigen Körpers (1011) erstreckt.

10. Verblindungskappe (1001, 2001, 3001, 4001) nach einem der vorhergehenden Ansprüche, bei der das erste Verriegelungselement (1070) an einer dem Scharnier (1030) gegenüberliegenden seitlichen Flanke (1017) des ersten langgestreckten schalenförmigen Körpers (1011) vorgesehen ist.

11. Verblindungskappe (1001, 2001, 3001, 4001) nach einem der vorhergehenden Ansprüche, bei dem das eine von erstem (1070) und zweitem (1090) Verriegelungselement über den ersten Rand (1015) hinausragt und das andere von erstem (1070) und zweitem (1090) Verriegelungselemente unter den zweiten Rand (1025) zurückgesetzt ist.

12. Verblindungskappe (1001, 2001, 3001, 4001) nach einem der vorhergehenden Ansprüche, bei der
das erste Verriegelungselement (1070) einen Verriegelungsrahmen mit einer Mehrzahl von Streben (1071) umfasst, die sich vom ersten Rand (1015) aus erstrecken und durch eine oder mehrere Verriegelungsleisten (1072) miteinander verbunden sind; und
das zweite Verriegelungselement (1090) eine oder mehrere Verriegelungslaschen (1091) umfasst, die konfiguriert sind, um durch Biegung der Streben (1071) mit den ein oder mehreren Verriegelungsleisten (1072) in Eingriff zu kommen.

13. Verblindungskappe (1001, 2001, 3001, 4001) nach einem der vorhergehenden Ansprüche, bei der der erste Teil (1010, 2010, 3010, 4010) und der zweite Teil (1020, 2020, 3020, 4020) undurchsichtig sind.

14. Spritzenanordnung (1200, 2200, 3200, 4200), umfassend eine Verblindungskappe (1001, 2001, 3001, 4001) nach einem der vorhergehenden Ansprüche und eine Spritze (1100), die einen Zylinder (1101) und eine starre Nadelabschirmung (1103, 2103, 3103, 4103) umfasst, die lösbar über einer Nadel der Spritze (1100) angebracht ist;
und optional, bei der die Spritze (1100) eine manuell betätigte Spritze ist.

## Revendications

1. Capuchon de protection (1001, 2001, 3001, 4001) pour recouvrir une protection d'aiguille rigide (1103, 2103, 3103, 4103) qui est montée, de manière amovible, sur une seringue (1100), le capuchon de protection (1001, 2001, 3001, 4001) comprenant une première partie (1010, 2010, 3010, 4010) et une seconde partie (1020, 2020, 3020, 4020) assemblées ensemble par une charnière (1030, 2030, 3030, 4030) ;
la première partie (1010, 2010, 3010, 4010) comprenant :
- un premier corps allongé en forme de cuvette (1011, 2011, 3011, 4011) définissant un premier évidement (1012) ;
- une première partie de bride (1040a) située au niveau d'une extrémité proximale (1014) ;
- un premier rebord (1015) s'étendant au moins partiellement autour d'une bouche ouverte du premier corps allongé en forme de cuvette (1011, 2011, 3011, 4011) ;
- une lèvre relevée (1050) s'étendant au moins partiellement le long du premier rebord (1015) ;
- une ou plusieurs ailettes (1060, 2060, 3060, 4060) qui font saillie vers l'intérieur à partir du premier corps allongé en forme de cuvette (1011, 2011, 3011, 4011) ; et
- un premier élément de verrouillage (1070) ;
la seconde partie (1020, 2020, 3020, 4020) comprenant :
- un second corps allongé en forme de cuvette (1021, 2021, 3021, 4021) définissant un second évidement (1022) ;
- une seconde partie de bride (1040b) située au niveau d'une extrémité proximale (1014) ;
- un second rebord (1025) s'étendant au moins partiellement autour d'une bouche ouverte du second corps allongé en forme de cuvette (1021, 2021, 3021, 4021) ;
- un évidement (1080) s'étendant au moins partiellement le long du second rebord (1025) ;
- une ou plusieurs ailettes (1061, 2061, 3061, 4061) qui font saillie vers l'intérieur à partir du second corps allongé en forme de cuvette (1021, 2021, 3021, 4021) ; et
- un second élément de verrouillage (1090) ;
dans lequel le capuchon de protection (1001, 2001, 3001, 4001) est configuré de sorte que la première partie (1010, 2010, 3010, 4010) et la seconde partie (1020, 2020, 3020, 4020) peuvent être amenés en mise en prise autour de la protection d'aiguille rigide (1103, 2103, 3103, 4103) par flexion de la charnière (1030, 2030, 3030, 4030) de sorte que :
i) la lèvre relevée (1050) de la première partie (1010, 2010, 3010, 4010) est mise en prise dans l'évidement (1080) de la seconde partie (1020, 2020, 3020, 4020) ;
ii) le premier élément de verrouillage (1070) et le second élément de verrouillage (1090) se mettent en prise entre eux afin de verrouiller la première partie (1010, 2010, 3010, 4010) et la seconde partie (1020, 2020, 3020, 4020) ensemble ;
iii) le premier évidement (1012) et le second évidement (1022) définissent ensemble une cavité pour recevoir la protection d'aiguille rigide (1103, 2103, 3103, 4103) de la seringue (1100, 2100, 3100, 4100) ;
iv) la première partie de bride (1040a) et la seconde partie de bride (1040b) sont juxtaposées pour former une bride en saillie vers l'intérieur qui est configurée pour être disposée entre une surface d'extrémité (1104) de la protection d'aiguille rigide (1103, 2103, 3103, 4103) et un corps cylindrique de la seringue (1100, 2100, 3100, 4100) ; et
v) les une ou plusieurs ailettes (1060, 2060, 3060, 4060, 1061, 2061, 3061, 4061) de la première partie (1010, 2010, 3010, 4010) et de la seconde partie (1020, 2020, 3020, 4020) sont configurées pour mettre en prise la protection d'aiguille rigide (1103, 2103, 3103, 4103).

2. Capuchon de protection (1001) selon la revendication 1, dans lequel les une ou plusieurs ailettes (1060, 1061) de la première partie (1010) et de la seconde partie (1020) définissent ensemble un alésage plus étroit à l'intérieur de la cavité pour mettre en prise la protection d'aiguille rigide (1103).

3. Capuchon de protection (1001, 2001, 3001, 4001) selon la revendication 1 ou la revendication 2, dans lequel les une ou plusieurs ailettes (2060, 4060, 2061, 4061) de la première partie (2010, 4010) et de la seconde partie (2020, 4020) comprennent une première partie (2060a, 4060a) définissant une butée d'extrémité pour une face d'extrémité (2103a, 4103a) de la protection d'aiguille rigide (2103) et/ou une seconde partie (2060b, 4060b) définissant une butée latérale pour une face latérale (2103b, 4103b) de la protection d'aiguille rigide (2103, 4103) et/ou une partie de coin définissant une butée de coin pour un coin (3103a) de la protection d'aiguille rigide (1103, 3103).

4. Capuchon de protection (1001, 2001, 3001, 4001) selon l'une quelconque des revendications précédentes, dans lequel les une ou plusieurs ailettes (1060, 2060, 3060, 4060, 1061, 2061, 3061, 4061) de la première partie (1010, 2010, 3010, 4010) et de la seconde partie (1020, 2020, 3020, 4020) comprennent une ou plusieurs ailettes flexibles ou une ou plusieurs ailettes rigides.

5. Capuchon de protection (1001, 2001, 3001, 4001) selon l'une quelconque des revendications précédentes, dans lequel la bride en saillie vers l'intérieur définit une ouverture proximale au niveau d'une extrémité proximale (1014).

6. Capuchon de protection (1001, 2001, 3001, 4001) selon l'une quelconque des revendications précédentes, dans lequel la bride en saillie vers l'intérieur comprend un bord chanfreiné.

7. Capuchon de protection (1001, 2001, 3001, 4001) selon l'une quelconque des revendications précédentes, dans lequel une extrémité distale (1013) de la première partie (1010) et de la seconde partie (1020) est arrondie de sorte que suite à la mise en prise de la première partie (1010) et de la seconde partie (1020), le capuchon de protection (1001, 2001, 3001, 4001) comprend une extrémité distale (1013) arrondie fermée.

8. Capuchon de protection (1001, 2001, 3001, 4001) selon la revendication 7, dans lequel la lèvre relevée (1050) s'étend autour de l'extrémité distale (1013) arrondie de la première partie (1010) et l'évidement (1080) s'étend autour de l'extrémité distale (1013) arrondie de la seconde partie (1020).

9. Capuchon de protection (1001, 2001, 3001, 4001) selon l'une quelconque des revendications précédentes, dans lequel la lèvre relevée (1050) s'étend le long d'un côté latéral (1016) du premier corps allongé en forme de cuvette (1011) à proximité de la charnière (1030).

10. Capuchon de protection (1001, 2001, 3001, 4001) selon l'une quelconque des revendications précédentes, dans lequel le premier élément de verrouillage (1070) est prévu sur un côté latéral (1017) du premier corps allongé en forme de cuvette (1011) opposé à la charnière (1030).

11. Capuchon de protection (1001, 2001, 3001, 4001) selon l'une quelconque des revendications précédentes, dans lequel l'un parmi le premier (1070) et le second (1090) élément de verrouillage fait saillie au-dessus du premier rebord (1015) et l'autre parmi le premier (1070) et le second (1090) élément de verrouillage est enfoncé au-dessous du second rebord (1025).

12. Capuchon de protection (1001, 2001, 3001, 4001) selon l'une quelconque des revendications précédentes, dans lequel :
le premier élément de verrouillage (1070) comprend un cadre de verrouillage comprenant une pluralité d'entretoises (1071) s'étendant à partir du premier rebord (1015) interconnecté par une ou plusieurs barres de verrouillage (1072) ; et
le second élément de verrouillage (1090) comprend une ou plusieurs languettes de verrouillage (1091) configurées pour se mettre en prise mutuellement avec les une ou plusieurs barres de verrouillage (1072) par flexion des entretoises (1071).

13. Capuchon de protection (1001, 2001, 3001, 4001) selon l'une quelconque des revendications précédentes, dans lequel la première partie (1010, 2010, 3010, 4010) et la seconde partie (1020, 2020, 3020, 4020) sont opaques.

14. Ensemble de seringue (1200, 2200, 3200, 4200) comprenant un capuchon de protection (1001, 2001, 3001, 4001) selon l'une quelconque des revendications précédentes, et une seringue (1100) qui comprend un corps cylindrique (1101) et une protection d'aiguille rigide (1103, 2103, 3103, 4103) qui est montée de manière amovible sur une aiguille de la seringue (1100) ;
et facultativement, dans lequel la seringue (1100) est une seringue activée manuellement.
